# EUROPEAN PATENT SPECIFICATION

(11) **EP 1 589 986 B1**
(45) Date of publication and mention of the grant of the patent: **29.10.2008**
(21) Application number: 03733748.2
(22) Date of filing: 16.06.2003
(51) Int. Cl.: A61K 38/17, G01N 33/68, A61P 9/10

(54) **METHODS AND USES OF THE INTEGRIN ALPHA 10 CHAIN, FOR DIAGNOSING AND DETECTING ATHEROSCLEROSIS PLAQUE FORMATION IN VITRO**
VERFAHREN UND VERWENDUNGSZWECKE DER INTEGRIN ALPHA 10 KETTE ZUR DIAGNOSE UND NACHWEIS VON ATHEROSKLEROTISCHER PLAQUEBILDUNG IN VITRO
METHODES ET UTILISATIONS DE LA CHAINE ALPHA 10 DE L'INTEGRINE, SERVANT A DIAGNOSTIQUER ET DEPISTER DE LA FORMATION DE PLAQUES D'ATHEROSCLEROSE IN VITRO

(30) Priority: 17.06.2002 SE 0201844; 17.06.2002 US 388747 P
(43) Date of publication of application: 02.11.2005
(73) Proprietor: Cartela R & D AB, 205 02 Malmö (SE)
(72) Inventor: LUNDGREN-AKERLUND, Evy, S-237 33 Bjärred (SE)
(74) Representative: Danfelter, Maria
(86) International application number: PCT/SE2003/001012
(87) International publication number: WO 2003/105886

(56) References cited:
- WO-A1-97/14432
- WO-A1-99/51639
- CHIA MARIE C.: 'The role of adhesion molecules in atherosclerosis' CRITICAL REVIEWS IN CLINICAL LABORATORY SCIENCES vol. 35, no. 6, 1998, pages 573 - 602, XP002962675
- CAMPER L.; HELLMAN U.; LUNDGREN-AKERLUND E.: 'ISOLATION, CLONING, AND SEQUENCE ANALYSIS OF THE INTEGRIN SUBUNIT ALPHA10, A BETA1-ASSOCIATED COLLAGEN BINDING INTEGRIN EXPRESSED ON CHONDROCYTES' JOURNAL OF BIOLOGICAL CHEMISTRY vol. 273, no. 32, 07 August 1998, pages 20383 - 20389

## Description

### TECHNICAL FIELD

This invention relates to methods and uses of the integrin alpha10 chain for detecting atherosclerotic plaque in a mammal *in vitro*.

### BACKGROUND OF THE INVENTION

### Atherosclerosis

Atherosclerosis and its thrombotic complications are the major cause of morbidity and mortality in industrialised countries. The number of prevalent atherosclerotic cases in 2000 totalled nearly 174 million in the major pharmaceutical markets and this figure will continue to increase with the increase in the ageing population. Still, only a fraction of these show overt signs of the disease. The rest remain undiagnosed until the disease manifests itself symptomatically, in the worst case as a heart attack or stroke.

Atherosclerosis is a focal pathological phenomenon characterised by the thickening and hardening of arteries due to the accumulation of lipids (mainly cholesterol esters), carbohydrates, blood products, fibrous tissue and calcium within the vessel wall beginning with the subendothelial space. The gradual build-up of fatty deposits leads to the formation of plaques, which eventually narrow and block the artery. The causes and detection of such plaque formation has been intensely investigated.

Atherosclerosis can occur in any artery. In coronary arteries, it may result in heart attacks; in cerebral arteries it may result in strokes; in peripheral arteries it may result in gangrene of the extremities.

The American Heart Association Committee on Vascular Lesions (Stary et al, Arterioscler Thromb. Vasc. Biol., 1995; 15 (9): 1512-31) has developed a standardised classification of distinct plaques simplified by Fuster (Fuster et al, Circulation, 1994 Oct; 90 (4): 2126-46) and reviewed by Corti et al (Ann N Y Acad Sci. 2001 ; 947: 181-95; and discussion on pages 195-8).

### The process of atherosclerosis

The process of atherosclerosis can be viewed as a special type of chronic inflammation where monocytes adhere to the vessel wall and accumulate in the intima. In the presence of low-density lipoproteins (LDL) the monocytes are converted to activated macrophages, which take up lipoprotein particles and become foam cells. This is followed by migration and proliferation of vSMCs (vascular smooth muscle cells) within the arterial intima, leading to the great intimal expansion seen in atherosclerotic plaques.

The proliferation of the vSMCs is concomitant with a phenotypic modulation of the vSMCs from a contractile to a synthetic phenotype. In addition to proliferation, this phenotypic modulation leads to an increase in the production of extracellular matrix (ECM) molecules (Thyberg et al, Arteriosclerosis. 1990;10(6):966-90). The ECM is critical for the maintenance of vascular integrity and imparts tensile strength, viscoelasticity, elastic recoil and compressibility through the distinct properties of its constituents. Interactions between the ECM and vSMCs are mediated via cell surface receptors such as integrins. Integrins are heterodimeric molecules consisting of non-covalently bound alpha and beta subunits. These molecules mediate a broad distribution of interactions between cells and extracellular matrix components and affect a number of cellular responses including adhesion, migration, proliferation, differentiation, and apoptosis.

### Alpha10

A newly discovered collagen-binding integrin, alpha10beta1, includes the integrin subunit alpha10 (Camper et al., (1998) J. Biol. Chem. 273:20383-20389). The integrin is expressed on chondrocytes and shows a Mᵣ of 160 kDa after reduction when isolated from bovine chondrocytes by collagen type II affinity purification.

Cloning and cDNA sequencing showed that it shares the general structure of other integrin alpha subunits. The predicted amino acid sequence consists of a 1167-amino acid mature protein, including a signal peptide (22 amino acids), a long extracellular domain (1098 amino acids) a transmembrane domain (22 amino acids), and a short cytoplasmic domain (22 amino acids). In contrast to most alpha-integrin subunits, the cytoplasmic domain of alpha10 does not contain the conserved sequence KXGFF(R/K)R. Instead, the predicted amino acid sequence in alpha10 is KLGFFAH. It is suggested that the GFFKR motif in alpha-chains are important for association of integrin subunits and for transport of the integrin to the plasma membrane (De Melker et al. (1997) Biochem. J. 328:529-537).

The extracellular part contains a 7-fold repeated sequence, an I-domain (199 amino acids) and three putative divalent cation-a binding site. Sequence analysis has revealed that the alpha10 subunit is most closely related to the I domain-containing α subunits with the highest identity to alpha1 (37%), alpha2 (35%) and alpha11 (42%).

### The role of integrins in atherosclerosis

Although several integrins are known to be present on normal vSMCs (Raines E. W. , Int J Exp Pathol. 2000 ; 81 (3): 173-82), little is known about the changes in integrin expression during atherosclerosis. It has been shown that injured (synthetic) vSMCs, in atherosclerosis, are able to modulate their integrin expression by switching from alphalbetal to alpha2betal and increase their production of fibrillar collagens, particularly collagen I (Skinner et al, Am J Pathol. 1994; 145 (5): 1070-81. ; Thyberg et al, Arteriosclerosis, 1990; 10 (6): 966-90).

Cellular interactions are critical for the development of the atherosclerotic lesion at all stages of its evolution. The integrins, which are receptors for ECM-molecules such as collagens, have critical roles in the vSMCs communication with the extracellular matrix and modulating the phenotype of the vSMCs (Hynes R. O., Cell. 1992; 69 (1) : 11-25). Future therapy will be directed towards the modulation of these adhesive interactions mediated by the integrins, which in turn may arrest the development of the atherosclerotic plaque, limit plaque activation and attenuate the thrombotic response accompanying activation.

To-date, treatment of atherosclerosis is mainly performed by dietary adjustments and administration of cholesterol-lowering drugs to slow down or even halter the development of atherosclerosis. However, only limited success has been reported for regression of atherosclerosis. i.e. diminution of the atherosclerotic fatty plaque deposits.

It is thus highly desirable in the light of the aforementioned problems to develop means and methods for suitable therapeutic target molecules and potential diagnostic markers in atherosclerosis, and especially for the inhibition and regression of atherosclerotic plaques. In this respect, the present invention addresses this need and interest.

### SUMMARY OF THE INVENTION

In view of the foregoing disadvantages known in the art when treating a mammal for atherosclerosis, or detecting atherosclerotic plaque, the present invention provides an antigen indicative of the presence of atherosclerotic plaque, namely the integrin alpha10 chain, suitable for detecting atherosclerosis *in vitro*.
One object with the present invention is to provide a method for detecting atherosclerotic plaque in a mammal *in vitro*.

Thus, the present invention provides a method for diagnosing a mammal who has or may be at risk of developing atherosclerosis, the method comprising the steps of
a) determining the amount of integrin alpha10 chain in a mammal *in vitro*,
b) scoring the amount of integrin alpha10 chain in said mammal, relative to a control
c) correlating the amount obtained in step b) above with amounts obtained from the control to determine whether the mammal has or is at risk of developing atherosclerosis.
wherein determining of the amount of integrin alpha10 chain further comprises contacting a biopsy from a part of the mammal where the atherosclerotic plaque is suspected to be located with a binding agent (as defined below) having a binding site specific for said of integrin alpha10 chain.

Even further, the present invention include a method for detecting atherosclerotic plaque in a mammal, the method comprising the steps of
a) determining the amount of integrin alpha10 chain in a mammal *in vitro* comprising contacting the integrin alpha10 chain with a binding agent (as defined below),
b) scoring the amount of integrin alpha10 chain in said mammal, relative to a control, and
c) correlating the amount obtained in step b) above with amounts obtained from the control to detect said atherosclerotic plaque in the mammal.

The methods according to the invention may in further embodiments include wherein the mammal is a human, or a rodent, such as a rabbit, mouse or rat, or a primate, such as chimpanzees, squirrel monkeys, howler monkeys, rhesus monkeys and cynomolgus monkeys, or a pig, e.g. the White Belgian pig.

Further embodiments include wherein the mammal is fed a high-cholesterol and/or high-fat diet.

Uses of the integrin alpha 10 chain for diagnosing atherosclerosis and for detecting atherosclerotic plaque *in vitro* are also provided.

### SHORT DESCRIPTION OF DRAWINGS

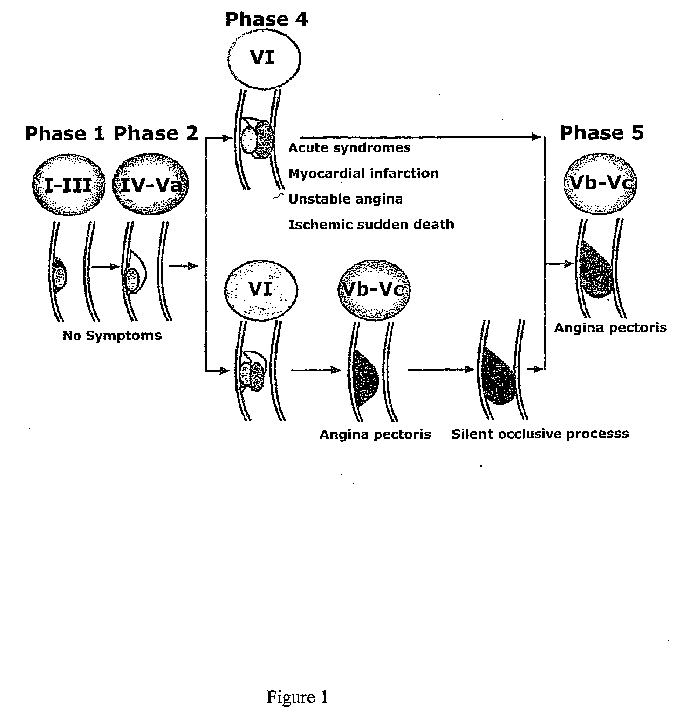

Fig. 1 shows phases and lesion morphology in the progression of atherosclerosis (from: Corti et al 2001 Ann. N. Y Acad. Sci 947: 181-198), and

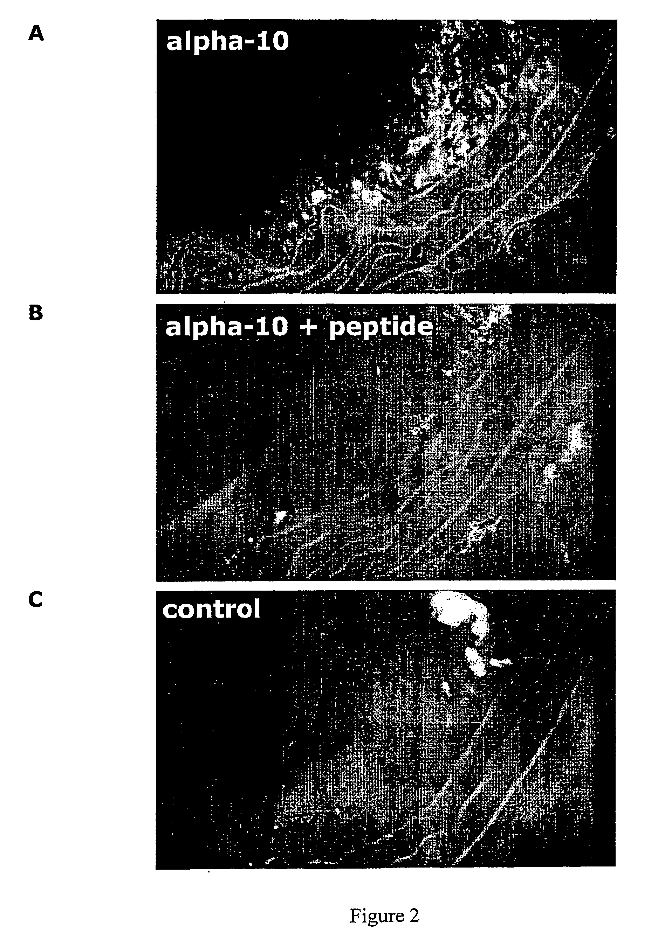

Fig. 2 shows the detection of integrin alpha10 chain in an atherosclerotic plaque of a murine aorta. The top picture, A, shows the staining of the integrin alpha10 chain as a strong staining of the cell surface of a cell in the aortic plaque. The middle photo, B, shows a specific blocking of the integrin alpha10 chain staining with a peptide of the integrin alpha10 chain, wherein almost all staining has been blocked by the peptide. The bottom photo, C, shows a control staining using secondary antibody only. No unspecific binding due to the secondary antibody, a donkey-anti-rabbit Cy3, is detected.

### DETAILED DESCRIPTION OF THE INVENTION

### Definitions

As used herein, the term"atherosclerotic plaque"refers to the build up of fatty plaque deposits within the wall of blood vessels.

The terms"rodent"and"rodents"refer to all members of the phylogenetic order Rodentia.

The term"murine"refers to any and all members of the family Muridae, including rats and mice.

A"therapeutically effective amount", or"effective amount", or "therapeutically effective", as used herein, refers to that amount which provides a therapeutic effect for a given condition and administration regimen. This is a predetermined quantity of active material calculated to produce a desired therapeutic effect in association with the required additive and diluent; i.e., a carrier, or administration vehicle. Further, it is intended to mean an amount sufficient to reduce and most preferably prevent, a clinically significant deficit in the activity, function and response of the host. Alternatively, a therapeutically effective amount is sufficient to cause an improvement in a clinically significant condition in a host. As is appreciated by those skilled in the art, the amount of a compound may vary depending on its specific activity. Suitable dosage amounts may contain a predetermined quantity of active composition calculated to produce the desired therapeutic effect in association with the required diluent; i.e., carrier, or additive.

As used herein,"treating", means treating for curing which may be a full curing or a partial curing of a condition or conditions associated with atherosclerosis.

As used herein"alleviation", means a decreased, i. e. less, or milder condition or conditions associated with atherosclerosis.

As used herein"preventing", means a complete or partial block of development, or outbreak, of a certain condition or conditions associated with atherosclerosis.

In the methods and use of the invention, a therapeutically effective amount of the active component or binding agent is provided. A therapeutically effective amount can be determined by the ordinary skilled medical or veterinary worker based on patient characteristics, such as age, weight, sex, condition, complications, other diseases, etc. , as is well known in the art.

### The antigen

As revealed above, the present invention provides an antigen indicative of the presence of atherosclerotic plaque, namely the integrin alpha10 chain sequence. The integrin alpha10 chain is known and publicly available at GenBank TM/EBI Data Bank accession number AF074015. Thus, new uses and methods of said integrin alpha10 chain are disclosed in the present invention.

Accordingly, the present invention provides the use of the integrin alpha10 chain as a therapeutic target molecule and potential diagnostic marker in atherosclerosis to meet today's unmet need for methods that can be used in prevention, early detection and therapy of atherosclerosis.

### The binding agent

The administration of an effective amount of a binding agent having a binding site specific for integrin alpha10 chain has to be evaluated in each case. The binding agent is an agent targeting integrin alpha 10, such as human integrin alpha10, or an RNA enclosing the same, selected from the group consisting of polyclonal or monoclonal antibodies, or parts thereof, or nucleic acids. The binding agent, when used *in vivo* as a diagnostic agent, has to be administered over a range of doses to the mammal in the need thereof, followed by assaying at various time points for (an) effect (s) of the agent on the atherosclerotic plaque. The effective dose of the binding agent can differ from mammal to mammal, and from patient to patient but in general includes amounts starting where diagnostic effects on the atherosclerotic plaque occur, but below that amount where significant undesirable side effects are observed. The dosage administered will depend on the age, health, and weight of the recipient, kind of concurrent treatment, if any, frequency of the treatment, and the nature of the effect desired. The effective dosage can be modified by comparing the relative to in vivo potencies of the drugs and the bioavailability using no more than routine experimentation.

The binding agent is generally administered locally and/or systemically together with a pharmaceutically acceptable carrier, diluents and/or other additives. Pharmaceutically acceptable carriers and/or diluents include any and all solvents, dispersion media, coatings, antibacterial and antifungal agents, isotonic and absorption delaying agents and the like. The use of such media and agents for pharmaceutical active substances is well known in the art. Except insofar as any conventional medium or agent is incompatible with the active ingredient, use thereof in the therapeutic compositions is contemplated. Supplementary active ingredients can also be incorporated into the compositions.

The binding agent in an effective amount may be administered in any convenient manner, either orally or parenteraly such as by intravenous, intraperitoneal, subcutaneous, rectal, implant, transdermal, slow release, intrabuccal, intracerebral or intranasal administration. For intracerebral administration, the binding agent needs to pass the blood brain barrier and may have to be chemically modified, e. g. made hydrophobic, to facilitate this or be administered directly to the brain or via other suitable routes.

For injectable use, sterile aqueous solutions, where water soluble, are generally used or alternatively sterile powders for the extemporaneous preparation of sterile injectable solutions may be used. It must be stable under the conditions of manufacture and storage and must be preserved against the contaminating action of microorganisms such as bacteria and fungi.

The carrier can be a solvent or dispersion medium containing, for example, water, ethanol, polyol, for example, glycerol, propylene glycol and liquid polyethylene glycol, and the like, suitable mixtures thereof, and vegetable oils. The prevention of the action of microorganisms can be brought about by various antibacterial and antifungal agents, for example, parabens, chlorobutanol, phenol, sorbic acid, thirmerosal and the like. In many cases, it will be preferable to include isotonic agents, for example, sugars or sodium chloride. Prolonged absorption of the injectable binding agent can be brought about by the use in the binding agent of agents delaying absorption, for example, aluminum monostearate and gelatin.

Sterile injectable solutions are prepared by incorporating the binding agent in the required amount in the appropriate solvent with various amounts of the other ingredients enumerated above, as required, followed by sterilization by, for example, filtration or irradiation. In the case of sterile powders for the preparation of sterile injectable solutions, the preferred methods of preparation are vacuum drying and the freeze-drying technique, which yield a powder of the active ingredient plus any additional desired ingredient from previously sterile-filtered solution thereof.

When the binding agents are suitably protected they may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. The amount of active binding agent is such that a suitable effective dosage will be obtained.

Different forms of tablets, troches, pills, capsules and the like may also be used and contain other components as listed below.

The binding agent may be administered together with other agents, such as drugs, proteins, i.e. antibodies and enzymes, or peptides, i.e. polypeptides or oligopeptides, known to effect atherosclerosis, and particularly the atherosclerotic plaques. Such an additional other agent may be administered directly or indirectly linked to the binding agent or non-linked.

The binding agent according to the invention may be a monoclonal or polyclonal antibody, or parts thereof, binding to the integrin alpha10 chain, or a part thereof, such as the I-domain or the splice domain. The integrin alpha10 chain may also be in a heterodimer formed with the integrin betal chain. Thus, the binding agent may bind to both of the integrin chains, i.e. the alpha10betal heterodimer.

In further embodiments, the binding agent may bind any intracellular or extra cellular part of the integrin alpha10 chain.
The binding agent may in further embodiments bind to the 1-domain and/or the splice domain of the integrin alpha 10 chain.

In further embodiments, the binding agent may bind to or hybridise any nucleic acid, such as ribonucleic acid, RNA, or deoxyribonucleic acid, DNA, encoding the integrin alpha10 chain.

Thus, the binding agent may be any isolated nucleic acid, e. g. a polynucleotide or oligonucleotide, such as ribonucleic acid (RNA) deoxyribonucleic acid (DNA), peptide nucleic acid (PNA), which hybridises to said DNA or RNA encoding an integrin subunit alpha10 and wherein said polyoligonucleotide or oligonucleotide fails to hybridise to a DNA or RNA encoding other integrins, such as the integrin subunit alpha11, alpha1, alpha2 e. t. c. This is a direct regulation of the alpha10 expression.

Atherosclerotic plaques are build-up in the intima of the arterial wall which comprising an endothelial monolayer on the luminal face with a subcellular elastic extracellular matrix containing a few smooth muscle cells. The fist sign of an atherosclerotic plaque is accumulation of lipoprotein in the intima. The lipoproteins attract monocytes which becomes macrophages that engulfs the lipoproteins (macrophage foam cells). This is followed by a migration and proliferation of smooth muscle cells into the intma, leading to a great thickening of the intima. This is also accompanied by a phenotypic modulation of the smooth muscle cells. Furthermore, lymphocytes can be seen in the plaques. The smooth muscle cells build-up an extracellular matrix cap that surrounds the lipids and inflammatory cells which is critical for the maintenance of vascular integrity. The plaque becomes a place for chronic inflammation that leads to fibrosis and further thickening of the intima (Stary HC et al. Circulation 1995; 92: 1355-1374, Fayad ZA et al. Circ Res 2001 ; 98: 305-316). The activated macrophages release several matrix degrading metalloproteases which leads to a degradation and instability of the extracellular cap of the plaque. When there is an unbalance between production and degradation of the extracellular matix and the degradation takes overhand there is an increased risk for plaque rupture. The integrin alpha10 chain has both an intracellular and an extracellular domain on the cells in the atherosclerotic plaques, such as smooth muscle cells, macrophages or endothelial cells.

Thus, the cell may be a smooth muscle cell and the binding site on the integrin alpha10 chain may be intracellular in and/or extracellular on the surface of such a cell. Functional modulations and numerical increases in intimal smooth muscle cells stand out among cellular changes in advanced human lesions. The smooth muscle cells are in part resident intimal cells that preceded the lesions and in part their progeny that arose as a response to various stimuli. The stimuli include lipid accumulation, disruption of intimal structure, damage to intimal cells and matrix, and deposits of platelets and fibrinogen, all of which may activate resident cells to produce mitogenic factors. Furthermore, the increase in collagen in the atherosclerotic lesions is produced by intimal smooth muscle cells (Stary HC et al. Circulation 1995 ; 92: 1355-1374).

In a specific embodiment, the binding site specific on the integrin alpha10 chain is on the cell surface of a smooth muscle cell.
The primary *in vivo* effect in a mammal is where the binding agent finds and localises the target, i.e. integrin alpha10 chain, on the cell surface of said cells in the atherosclerotic plaque. Still, for detecting an atherosclerotic plaque in vitro or in vivo, as described below, the intracellular expression of integrin alpha10 chain may be used as well.

### A method for diagnosing a mammal who has or may be at risk of developing atherosclerosis in vitro

According to the summary of the invention, a method for diagnosing a mammal who has or is at risk of developing atherosclerosis is included. The method comprises the steps of
a) determining the amount of integrin alpha10 chain in a mammal *in vitro*,
b) scoring the amount of integrin alpha10 chain in said mammal, relative to a control, and
c) correlating the amount obtained in step b) above to determine whether the mammal has or is at risk of developing atherosclerosis.

In further embodiments, the method is a quantitative method, wherein the amount of integrin alpha10 chain is determined relative to the control in a quantitative manner.

An antibody or a fragment thereof binding specifically to the integrin alpha10 chain, unlabelled or labelled with an agent such as biotin, a fluorescent probe or an isotope may be used. A polypeptide binding specifically to the integrin alpha10 chain may be e.g. parts of the antigen binding site of an antibody binding to the integrin alpha10 chain. Said protein will be detected either directly or used to direct a contrast agent, such as gadolinium to the atherosclerotic plaque. Bound antibody or contrast agent may be visualized using techniques such as high-resolution magnetic resonance imaging (MRI), ultrasound, fluorescence emission spectroscopy or nuclear imaging. Any other suitable method known to the skilled man in the art may also be used. The amount of bound contrast agent, which can be quantified using techniques mentioned above, will, thus, reflect the amount integrin alpha10 chain in the tissue (Naghavi M et al.. Current Atherosclerosis Reports 2001; 3: 125-135. Yu X et al. Magnetic Resonance in Medicine 2000 ; 44: 867-872. Flacke S et al. Circulation 2001 ; 104: 1280-1285. Tsimikas S et al. Arterosler. Thromb. Vasc. Biol. 2000; 20: 289-697).

The amount of the integrin alpha 10 chain is determined in a biopsy from the mammal, such as a mouse, rat or human.

For example the biopsy may be from any blood vessel, such as an artery.

Determining of the amount of integrin alpha10 chain further comprises contacting atherosclerotic plaque, or part of the mammal where the atherosclerotic plaque is suspected to be located, with a binding agent having a binding site specific for said integrin alpha10 chain. Said binding agent is further described above.

Even further embodiments include wherein the scoring in b) above and correlating in c) above is a scoring and correlation to a particular disease stage of atherosclerosis. Different ways of scoring atherosclerotic plaque are known and outlined in brief below.

The Committee on Vascular Lesions has attempted to correlate the appearance of lesions noted in clinical imaging studies with histological lesion types and corresponding clinical syndromes. In the histological classification, lesions are designated by Roman numerals, which indicate the usual sequence of lesion progression as outlined in figure 1. The initial (type I) lesion contains enough atherogenic lipoprotein to elicit an increase in macrophages and formation of scattered macrophage foam cells. Type II lesions consist primarily of layers of macrophage foam cells and lipid-laden smooth muscle cells and include lesions grossly designated as fatty streaks. Type III is the intermediate stage between type II and type IV (atheroma, a lesion that is potentially symptom-producing). In addition to the lipid-laden cells of type II, type III lesions contain scattered collections of extracellular lipid droplets and particles that disrupt the coherence of some intimal smooth muscle cells. This extracellular lipid is the immediate precursor of the larger, confluent, and more disruptive core of extracellular lipid that characterizes type IV lesions. Beginning around the fourth decade of life, lesions that usually have a lipid core may also contain thick layers of fibrous connective tissue (type V lesion) and/or fissure, hematoma, and thrombus (type VI lesion). Some type V lesions are largely calcified (type Vb), and some consist mainly of fibrous connective tissue and little or no accumulated lipid or calcium (type Vc) (Stary HC et al. Circulation 1995; 92: 1355-1374).

According to the criteria of the American Heart Association Committee on Vascular lesions, lesion progression can be divided into five phases as outlined in figure 1. The progression is based on pathological and clinical findings. In phase 1, early lesions are found (lesions type I-III). These lesions can then progress into phase 2 with fibrolipid plaques (lesions type IV-Va). Phase 2 can progress into an acute phase (phase 3 or 4) and phase 3 and 4 lesions are of type VI. Formation of thrombosis or hematoma may cause angina pectoris (phase 3) or acute coronary syndrome due to occlusive thrombosis (phase 4). Phase 3 and 4 lesions can evolve into a fibrotic phase (phase 5) characterized by more stenotic plaques (lesions type Vb-c) that may progress to occlusive lesions (Fayad ZA et al. Circ Res 2001; 98 : 305-316).

A method for detecting atherosclerotic plaque in a mammal *in vitro*

According to the summary of the invention, a method for detecting atherosclerotic plaque in a mammal is disclosed. Such a method comprises the steps of
a) determining the amount of integrin alpha10 chain in a mammal *in vitro*,
b) scoring the amount of integrin alpha10 chain in said mammal, relative to a control, and
c) correlating the amount obtained in step b) above with amounts obtained from a control to detect the amount of atherosclerotic plaques.

In further embodiments, the method is a quantitative method, wherein the amount of integrin alpha10 chain in a mammal is determined relative to the control sample in a quantitative manner.

In even further embodiments, the amount of the integrin alpha10 chain is determined in a biopsy from the mammal, such as a mouse, rat or human.

Further embodiments include wherein the biopsy is from any blood vessel, such as an artery.

Further embodiments include wherein the amount of integrin alpha10 chain is determined in vitro in a biopsy from said mammal, such as a mouse, rat or human.

Further embodiments include wherein the determining of the amount of integrin alpha10 chain is performed by immunohistochemical analysis in vitro. Such methods are known to the skilled man in the art, and further exemplified in the example below.

In brief, the tissues are fixed by fixative or frozen using methods known to the skilled man in the art. After the tissues are fixed, they may be embedded in into paraffin or mounted in Tissue Tek^{®} O. C. T. compound (Sakura Finetek Europe B. V., The Netherlands) and frozen for further sectioning. Paraffin-embedded tissues are cut using e. g. a microtome at room temperature, whereas frozen tissues are cut using a cryostat at temperatures below 0°C. Sections are then treated in a way that best retrieve the antigen several methods are available and known to the skilled man in the art. Furthermore, the tissue sections may be blocked for any endogenous antigens or staining that can be detected by the detection system as background. The sections are then incubated with antibodies specific to integrin alpha10 chain. The antibody may be labelled with agents such as fluorescence, enzyme or biotin that then are detected with suitable detection system. Alternatively, a secondary antibody, labelled with agents such as fluorescence, enzyme or biotin may be used to detect the anti-integrin alpha10 chain antibody. The amount of staining may the be measured using computerized imaging systems. The amount of staining correlates to the amount of alpha10 chain in the tissue. (Camper L et al Cell Tissue Res. 2001; 306: 107-116).

Also described herein is determination of the amount of integrin alpha10 chain by any invasive or non-invasive method in vivo, such as any imaging method. Examples of such methods are Magnetic Resonance Imaging (MRI), Ultrasound such as intravascular ultrasound (IVUS), Computed tomography, such as Electron Beam Computed Tomography (EBCT) and multislice tomographic scanning, as well as angiography. Any other suitable method known to the skilled man in the art may also be used.

Even further embodiments include wherein determining of the amount of integrin alpha10 chain further comprises contacting the sample with a binding agent having a binding site specific for said integrin alpha10 chain. Said binding agent is further described above.

### A method for modulating the expression of the integrin alpha10 chain.

The expression of the integrin alpha10 may also be modulated by gene targeting of the gene(s) encoding the alpha10 protein expression in a mammalian cell. The cell to be targeted may be targeted in vitro or in vivo in a mammal, such as a human or a mouse. In vitro techniques for modulating expression of a specific gene, or group of genes are known to a person skilled in the art. Examples are given in Talts JF. , Brakebusch C., Fassler, R (1999) Integrin gene targeting. Methods Mol. Biol., 129,153-187, Kozarsky KF (2001) Current Opinion in Pharmacology 1: 1997- 202).

In brief, a gene is normally targeted by a construct encoding a complementary nucleic acid sequence to the gene of interest to target. The whole gene may be targeted, as well as a part thereof. "A part thereof here refers to a part of the gene that will regulate the expression directly or indirectly, such as the promotor or other regulatory sequences of the gene, as well as the intron or exon sequences of the gene. Thus, the expression of the alpha10 protein may, due to the targeting at the gene level, be modulated to e. g. down-regulate or up-regulate the expression of the alpha10 integrin protein.

Described herein is a method for modulating the expression of the integrin alpha10 chain in a cell in an atherosclerotic plaque. Such a method comprises the steps of :
a) providing a construct targeting the integrin alpha10 chain gene, parts thereof or regulatory sequences to the gene,
b) contacting the cell expressing the integrin in an atherosclerotic plaque in vivo or in vitro with said construct,
c) transforming the cell in vivo or in vitro,
d) optionally selecting the transformed cell in vivo or in vitro,
e) detecting the protein expression ofthe alpha10 chain protein,
f) correlating the protein expression of the alpha 10 chain protein to the expression before providing the construct in a) above, thereby evaluating the modulation of the expression of the integrin alpha10 chain protein.

Modulating the expression of the integrin alpha10 chain may be done directly or indirectly through other factors that control the integrin alpha10 protein expression. Examples of factors are growth factors such as fibroblast growth factor, insulin-like growth factor, transforming growth factor-beta, parathyroid hormone-related peptide, bone morphogenic proteins, Indian hedgehog and sonic hedgehog; transcription factors such as SOX5-6 and SOX9 or other factors that modulates the expression of integrin alpha10 protein.

A construct may comprise any agent that targets the integrin alpha10 chain gene, parts thereof or regulatory sequences to the gene. It may also target genes, parts thereof or regulatory sequences to genes of factors that modulate the expression of the integrin alpha10 protein.

Furthermore, the construct may comprise a viral or non-viral system for the in vivo delivery of any of the genes, parts thereof or regulatory sequences to the genes described above. Examples of viral vectors include retroviruses, adenoviruses, adenoviruses, adeno-associated viruses (AAV), herpes simplex virus and lentivirus. Examples of non-viral delivery systems include the use of naked DNA, cationic liposomes, cationic lipids and polymers as well as DNA/cationic liposome/polycation complexes.

The expression of the exogenous genetic material in vivo is often referred to as "gene therapy". Disease states and procedures for which such treatments and methods according to the invention have application include genetic disorders and diseases of the vascular system such as atherosclerosis. Cell delivery of the transformed cells may be effected using various methods and includes infusion. One example of an infusion method is described by Tangirala RK et al. (1999) Circulation 100: 1816-1822.

A construct may further include a chemical compound that targets the integrin alpha10 chain gene, parts thereof or regulatory sequences to the gene. It may also target genes, parts thereof or regulatory sequences to genes of factors that modulate the expression of the integrin alpha10 protein, as described above.

Anti-sense technique may be used to modulate the transcription of the genes, mentioned above, that in the end affects the expression of integrin alpha10 chain. The construct could include anti-sense DNA or RNA, i.e. DNA or RNA oligonucleotids that are complimentary to a given mRNA sequence of interest. The binding of the DNA and RNA oligonucleotids to the mRNA will block the translation of that particular mRNA.

Examples of delivery systems for anti-sense include the use of naked DNA or RNA, cationic liposomes, cationic lipids and polymers as well as DNA/cationic liposome/polycation complexes.

Furthermore, the construct may include a viral vector for the in vivo delivery of any of the genes, parts thereof or regulatory sequences to the genes described above. Examples of viral vectors include retroviruses, adenoviruses, adenoviruses, adeno-associated viruses (AAV), herpes simplex virus and lentivirus.

Further approaches include wherein the targeting of the gene is modulating the expression so as to up-regulate the expression of the alpha10 chain protein. The up-regulation may, thus, be to a normal level of the gene. A "normal level" is herein intended to mean a level about what the cell in a specific mammal normally expresses in that particular cell type and activation stage of that cell type. This is sometimes referred to as the wild type expression as well.

Even further approaches include in vivo modulation of the integrin alpha10 chain protein expression. Such *in vivo* modulation may as well be due to a regulation at the gene level due to targeting of the gene in vivo. Thus, the expression of the alpha10 protein may, due to the targeting at the gene level, be modulated to e.g. down regulate the expression of the protein *in vivo*.

Further approaches include wherein the targeting of the gene is modulating the expression so as to up-regulate the expression of the aplha10 chain protein *in vivo*.

The method above may be used as a gene therapeutic method for modulating the expression of the integrin alpha10 chain protein. Such a use may be to suppress, prevent, increase or decrease the in vivo expression in a mammal, such as a human, mouse or rat, in the need thereof. By re-introduction of nucleic acid, such as deoxynucleic acid (DNA), ribonucleic acid (RNA), peptide nucleic acid (PNA) or mixtures thereof, the activity may be modulated and e. g. an absence or over-expression of the integrin alpha10 chain protein partially or fully compensated.

Still another use of the method described herein is to prevent, inhibit, alleviate or reverse activity in atherosclerosis.

### The mammal

For the methods and uses disclosed, a mammal is considered. In further embodiments, the mammal may be a human being. Still further embodiments include wherein the mammal is a rodent, such as a rat or mouse or any other member of the family Muridae.

Further embodiments include wherein the mammal is a rabbit, or a primate, such as chimpanzees, squirrel monkeys, howler monkeys, rhesus monkeys and cynomolgus monkeys, or a pig, e. g. the White Belgian pig.

Further embodiments include wherein the mammal is fed a high-cholesterol and/or high-fat diet.

In some of the methods and uses, a mammal in the need thereof is disclosed. Such a mammal, e.g. a human or mouse, may be any mammal showing early signs of atherosclerotic plaque, fully developed atherosclerotic plaque, or being predisposed to or in the risk of forming an atherosclerotic plaque.

Different conditions may lead to atherosclerosis in a mammal. Thus, further embodiment include wherein the atherosclerosis in a mammal is caused by conditions such as type 1 diabetes, ApoAl deficiency, a cardiovascular disorder, e. g. coronary artery disease (CAD), systemic lupus erythematosis (SLE), acute ischemia, chronic ischemia or Sjögren's syndrome.

Other risk factors for atherosclerosis include hypertension, elevated levels of low density lipoprotein, reduced levels of high density lipoprotein, cigarette smoking, diabetes mellitus, obesity, male sex, elevated homocysteine levels, chlamydia pneumoniae infection, and family history of premature atherosclerosis. Thus, even further embodiments include wherein the mammal in the need thereof is a mammal being exposed to different risk factors involved in atherosclerosis, such as the risk factors mentioned above.

### Uses according to the invention

According to the invention, several uses of the integrin alpha10 chain are disclosed.

A use of integrin alpha10 chain for diagnosing atherosclerosis *in vitro* is disclosed.

Even further, a use of integrin alpha10 chain for detecting atherosclerotic *in vitro* plaque is included.

### EXAMPLES

### Example 1 Detection of the integrin alpha10 chain in aortic atherosclerotic plaque

### Objective

The objective of this example is to describe detection of the integrin alpha10 chain in aortic atherosclerotic plaque.

### Material

Murine aortic plaque sections were used. The sections were cut on a cryostat at a thickness of 5, um onto Superfrost Plus slides (Menzel-Glaser, Histolab). The cut sections were then stored at-20°C.

### Detection of the aortic plaques

Before staining, the frozen sections were allowed to thaw to room temperature for about 30 minutes.

The sections were fixed in acetone for 10 minutes at -20°C, and then treated with hyaluronidase (2mg/ml, Sigma) for 30 minutes at 37°C.

The sections were blocked with 4% donkey serum (Harlan) diluted in PBS for 30 minutes at room temperature.

During the blocking step, an anti-alpha10 antibody was incubated with an alpha10 peptide, i.e. the blocking peptide, at different concentrations, e.g. 0.01, 0.1, and 0.5 mg/ml, for 30 minutes at 4°C.

The sections were then incubated with rabbit anti-human alpha 10, in 1:100 dilution, and a fraction of the preincubated rabbit anti-human alpha10 and the blocking peptide in a 1:100 dilution. The sections were incubated for 75 minutes at room temperature.

After a 15-minute wash in PBS, the sections were incubated with donkey anti-rabbit Cy3 (IgG, 1:100, from Jackson) for 60 minutes in room temperature.

After a final 15-minute wash in PBS, the sections were mounted with Vectashield (Vector Labs), and photographed under a fluorescent microscope.

### Results and discussion

In figure 2, the result of the staining to detect the integrin alpha10 expression in the aortic plaques is shown. The top picture, A, shows the staining of the integrin alpha10 chain as a strong staining around the outside of the cells in the aortic plaque. The middle photo, B, shows a specific blocking of the integrin alpha10 chain staining with a peptide of the integrin alpha10 chain, wherein almost all staining has been blocked by the peptide. The bottom photo, C, shows a control staining with the secondary antibody used. No unspecific binding due to the secondary antibody, a donkey-anti-rabbit Cy3, is detected.

### Example 2 Evaluating the involvement of integrin alpha10 chain in neointima formation following vascular injury in integrin alpha10 chain deficient mice

### Objective

The objective of this example is to evaluate the role and function of integrin alpha10 chain in the formation of neointima (or atherosclerotic plaque) and cholesterol levels following vascular injury.

### Animals

Integrin chain alpha10 ^{-/-} mice and wild-type age and gender matched control mice.

### Mouse artery injury

Several methods for mechanically induced artery injury on mice are available and known to the skilled man in the art. One example, carotid cuff-injury, is presented here (Dimayuga P et al, Biochemical and Biophysical Research Communications, 1999; 264:465-468).

The animals are anesthetized with isoflurane followed by Avertin (intraperitoneal injection of 0.016 ml/g bodyweight of a 2.5% solution).

The right carotid artery is carefully isolated under a dissection microscope.

A 2.5 mm long tube is applied around the carotid artery and the skin incision is closed.

### Analysis

The size of the neointima will be measured and its content with respect to cells such as inflammatory cells, smooth muscle cells, fibroblasts and endothelial cells and extracellular matrix components such as collagens, proteoglycans and fibronectin and different cell surface markers such as integrins will be analysed using different staining techniques and specific antibodies known to the skilled man in the art.

Mice are killed 3 weeks after the induced injury.

The carotid arteries are carefully dissected out and perfused with 0.9% saline, embedded in Tissue Tek^{®} O.C.T. compound (Sakura Finetek Europe B.V., The Netherlands) and frozen in -70°C or alternatively fixated in phosphate-buffered formalin and embedded in paraffin.

Sections of the tissue are then stained with hematoxylin and eosin staining and /or Verhoeff Van-Giesion staining for morphological studies. Morphometric analyses will be performed with Verhoeff Van-Gieson-stained sections.

Measurements of luminal area, area inside the internal elastic lamina (IELA), and the area encircled by external elastic lamina will be made on each section. Medial area will be calculated by subtracting IELA from external elastic lamina, and intimal area (IA) will be calculated by subtracting luminal area from IELA. From these measurements, the ratio of IA and medial area (I/M ratio) will be calculated. Computer assisted image analysis will be used for quantitative assessment of stainings and for morphometric analyses of the neointima (systems such as Optimas System, Bioscan or Scion Image analysis software, Scion) (Dimayuga P et al, Biochemical and Biophysical Research Communications, 1999; 264:465-468, Zhu et al, Arterioscler Thromb Vasc Biol 2002; 22:450-455).

Plasma samples will be collected for measurements of total cholesterol levels using an enzymatic assay (Sigma, St Louis, MO, U.S.A.) HDL levels will be measured after precipitating apo-B containing lipoproteins with phosphotungstic acid (Sigma, St Louis, MO, U.S.A.).

Also levels of tissue cholesterol will be measured as described in (Dimayuga P et al, Biochemical and Biophysical Research Communications, 1999; 264:465-468).

### Example 3 Evaluating the involvement and function of integrin alpha10 chain in development atherosclerosis in integrin chain alpha 10^{-/-} apoE^{-/-} mice

### Objective

The objective with this example is to create a double knock-out mouse, deficient for both the integrin alpha10 chain and apoE (alpha10^{-/-} apoE^{-/-}).

One further objective is to compare the alpha10^{-/-} apoE^{-/-} with age and gender matched single knock-out mice (alpha10^{-/-} and apoE^{-/-}) and wild-type control mice with respect to the development of atherosclerosis and cholesterol levels.

### Animals

Integrin chain alpha10^{-/-}, apoE ^{-/-} mice and age and gender matched wild-type controls. The mice will be fed standard chow diet (such as PicoLab Rodent chow 20, 4,5% fat by weight, 0.02% cholesterol) or Western-type diet (such as Tekland Adjusted Calories Western-type diet, 21% fat by weight, 0.15% cholesterol, 19.5% casein without sodium cholate).

### Generation of alpha10^{-/}⁻ apoE^{-/}⁻ double knock-out mice

Homozygous integrin chain alpha 10^{-/-} mice will be mated with homozygous apoE^{-/-} mice. The generated heterozygous F 1 mice will further be used to generate mice homozygous for both alpha10 and apoE deficiency.

### Analysis of atherosclerosis development in the integrin chain alpha10^{-/}⁻ and apoE^{-/}⁻ double knock-out mice

The size of the neointima will be measured and its' content with respect to cells such as inflammatory cells, smooth muscle cells, fibroblasts and endothelial cells and extracellular matrix components such as collagens, proteoglycans and fibronectin and different cell surface markers such as integrins will be analysed using different staining techniques and specific antibodies known to the skilled man in the art.

Mice will be killed at different ages to investigate the progression of the atherosclerosis.

The arteries are carefully dissected out and perfused with 0.9% saline, embedded in Tissue Tek® O.C.T. compound (Sakura Finetek Europe B.V., The Netherlands) and frozen in -70°C or alternatively fixated in phosphate-buffered formalin and embedded in paraffin.

Sections of the tissue are then stained with hematoxylin and eosin staining and /or Verhoeff Van-Giesion staining for morphological studies.

Morphometric analyses will be performed with Verhoeff Van-Gieson-stained sections.

Measurements of luminal area, area inside the internal elastic lamina (IELA), and the area encircled by external elastic lamina will be made on each section. Medial area will be calculated by subtracting IELA from external elastic lamina, and intimal area (IA) will be calculated by subtracting luminal area from IELA. From these measurements, the ratio of IA and medial area (I/M ratio) will be calculated. Computer assisted image analysis will be used for quantitative assessment of stainings and for morphometric analyses of the neointima (systems such as Optimas System, Bioscan or Scion Image analysis software, Scion) (Dimayuga P et al, Biochemical and Biophysical Research Communications, 1999; 264:465-468, Zhu et al, Arterioscler Thromb Vasc Biol 2002; 22:450-455).

Plasma samples will be collected for measurements of total cholesterol levels using an enzymatic assay (Sigma, St Louis, MO, U.S.A.) HDL levels will be measured after precipitating apo-B containing lipoproteins with phosphotungstic acid (Sigma, St Louis, MO, U.S.A.).

Also levels of tissue cholesterol will be measured as described in (Dimayuga P et al, Biochemical and Biophysical Research Communications, 1999; 264:465-468).

Results from the integrin chain alpha 10^{-/-}, apoE ^{-/-} double knock-out mice will be compared with age and gender matched single knock-out mice (integrin chain alpha 10^{-/-} and apoE ^{-/-} knock-out mice) and wild-type control mice.

## Claims

1. A method for diagnosing a mammal who has or may be at risk of developing atherosclerosis, the method comprising the steps of
a) determining the amount of integrin alpha10 chain in a mammal *in vitro,*
b) scoring the amount of integrin alpha10 chain in said mammal, relative to a control,
c) correlating the amount obtained in step b) above with amounts obtained from the control to determine whether the mammal has or is at risk of developing atherosclerosis,
wherein determining the amount of integrin alpha10 chain in a mammal comprises contacting a biopsy from a part of the mammal where the atherosclerotic plaque is suspected to be located with a binding agent having a binding site specific for said integrin alpha10 chain, or RNA encoding the same, selected from the group consisting of polyclonal or monoclonal antibodies, or parts thereof, or nucleic acids.

2. The method according to claim 1 wherein the biopsy is from a blood vessel.

3. The method according to claim 1 or 2 wherein the binding agent is a polyclonal or monoclonal antibody, or part thereof.

4. The method according to claim 1 or 2 wherein the binding agent is a nucleic acid.

5. A method for detecting atherosclerotic plaque in a mammal, the method comprising the steps of
a) determining the amount of integrin alpha10 chain in a mammal *in vitro,*
a) scoring the amount of integrin alpha10 chain in said mammal, relative to a control, and
b) correlating the amount obtained in step b) above with amounts obtained from the control to detect said atherosclerotic plaque in the mammal,
wherein determining the amount of integrin alpha10 chain further comprises contacting integrin alpha10 chain with a binding agent having a binding site specific for said integrin alpha10 chain, or RNA encoding the same, selected from the group consisting of polyclonal or monoclonal antibodies, or parts thereof, or nucleic acids.

6. The method according to claim 5 wherein the binding agent is a polyclonal or monoclonal antibody, or part thereof.

7. The method according to claim 5 wherein the binding agent is a nucleic acid.

8. The methods according to any of claims 5-7, wherein the mammal is a human.

9. The methods according to any of claims 5-7, wherein the mammal is a mouse.

10. Use of integrin alpha10 chain as a diagnostic marker for detecting atherosclerotic plaque *in vitro.*

11. Use of a binding agent having a binding site specific for said integrin alpha10 chain, or RNA encoding the same, in the preparation of a diagnostic agent for detecting atherosclerotic plaque in a mammal, wherein the binding agent is selected from the group consisting of polyclonal or monoclonal antibodies, or parts thereof, or nucleic acids.

12. The use according to claim 11 wherein the binding agent is a polyclonal or monoclonal antibody, or part thereof.

13. The use according to claim 11 wherein the binding agent is a nucleic acid.

14. The use according to, any one of claims 11 to 13 wherein the mammal is a human.

15. The use according to any one of claims 11 to 13 wherein the mammal is a mouse.

## Patentansprüche

1. Verfahren zur Untersuchung eines Säugers, der Arteriosklerose hat oder das Risiko läuft, eine solche zu entwickeln, wobei das Verfahren folgende Schritte umfasst
a) in-vitro-Bestimmung der Menge an Integrin-Alpha10-Kette in dem Säuger,
b) Auswerten der Menge von Integrin-Alpha10-Kette in dem Säuger bezogen auf eine Kontrolle,
c) Korrelieren der in Stufe b) oben erhaltenen Menge mit Mengen, die von der Kontrolle erhalten wurden, um zu bestimmen, ob der Säuger Arteriosklerose hat oder das Risiko läuft, eine solche zu entwickeln,
worin das Bestimmen der Menge der Integrin-Alpha10-Kette in einem Säuger ein Inkontaktbringen einer Biopsie von einem Teil des Säugers, wo die arteriosklerotischen Ablagerungen vermutlich liegt, mit einem Bindemittel mit einer für die Integrin-Alpha10-Kette oder diese kodierende RNA spezifischen Bindungsstelle, welche aus der Gruppe ausgewählt ist, bestehend aus polyclonalen oder monoclonalen Antikörpern oder Teilen davon oder Nukleinsäuren.

2. Verfahren gemäß Anspruch 1, worin die Biopsieprobe von einem Blutgefäß stammt.

3. Verfahren gemäß Anspruch 1 oder 2, worin das Bindemittel ein polyclonaler oder monoclonaler Antikörper oder ein Teil davon ist.

4. Verfahren gemäß Anspruch 1 oder 2, worin das Bindemittel eine Nukleinsäure ist.

5. Verfahren zur Feststellung von arteriosklerotischen Ablagerungen in einem Säuger, wobei das Verfahren die folgenden Schritte umfasst
a) in-vitro-Bestimmung der Menge an Integrin-Alpha10-Kette in einem Säuger,
b) Auswerten der Menge an Integrin-Alpha10-Kette in dem Säuger, bezogen auf eine Kontrolle und
c) Korrelieren der in Stufe b) oben erhaltenen Menge mit Mengen, die von der Kontrolle erhalten wurden, um die arteriosklerotische Ablagerung in dem Säuger festzustellen,
worin das Bestimmen der Menge der Integrin-Alpha10-Kette des Weiteren ein Inkontaktbringen der Integrin-Alpha10-Kette mit einem Bindemittel umfasst, welches eine für die Integrin-Alpha10-Kette oder diese kodierende RNA spezifische Bindungsstelle aufweist, ausgewählt aus der Gruppe, bestehend aus polyclonalen oder monoclonalen Antikörpern oder Teilen davon oder Nukleinsäuren.

6. Verfahren gemäß Anspruch 5, worin das Bindemittel ein polyclonaler oder monoclonaler Antikörper oder ein Teil davon ist.

7. Verfahren gemäß Anspruch 5, worin das Bindemittel eine Nukleinsäure ist.

8. Verfahren gemäß einem jeden der Ansprüche 5 bis 7, worin der Säuger ein Mensch ist.

9. Verfahren gemäß einem jeden der Ansprüche 5 bis 7, worin der Säuger eine Maus ist.

10. Verwendung der Integrin-Alpha10-Kette als Diagnosemarker zur in-vitro-Feststellung von arteriosklerotischen Ablagerungen.

11. Verwendung eines Bindemittels mit einer für die Integrin-Alpha10-Kette oder diese kodierende RNA spezifischen Bindungsstelle, bei der Herstellung eines Diagnosemittels zur Feststellung arteriosklerotischer Ablagerung in einem Säuger, wobei das Bindemittel aus der Gruppe ausgewählt ist, bestehend aus polyclonalen oder monoclonalen Antikörpern oder Teilen davon oder Nukleinsäuren.

12. Verwendung gemäß Anspruch 11, worin das Bindemittel ein polyclonaler oder monoclonaler Antikörper oder Teil davon ist.

13. Verwendung gemäß Anspruch 11, worin das Bindemittel eine Nukleinsäure ist.

14. Verfahren gemäß einem jeden der Ansprüche 11 bis 13, worin der Säuger ein Mensch ist.

15. Verfahren gemäß einem jeden der Ansprüche 11 bis 13, worin der Säuger eine Maus ist.

## Revendications

1. Procédé pour diagnostiquer un mammifère qui présente ou peut être exposé à un risque de développer de l'athérosclérose, le procédé comprenant les étapes consistant à
a) déterminer la quantité de chaîne alpha 10 de l'intégrine chez un mammifère *in vitro,*
b) attribuer un score à la quantité de chaîne alpha 10 de l'intégrine chez ledit mammifère, par rapport à un contrôle,
c) corréler la quantité obtenue à l'étape b) ci-dessus avec des quantités obtenues à partir du contrôle afin de déterminer si le mammifère présente ou est exposé à un risque de développer de l'athérosclérose,
où la détermination de la quantité de chaîne alpha 10 de l'intégrine chez un mammifère consiste à mettre en contact une biopsie prélevée à partir d'une partie du mammifère où l'on suspecte la présence d'une plaque d'athérosclérose avec un agent de liaison ayant un site de liaison spécifique pour ladite chaîne alpha 10 de l'intégrine, ou pour un ARN codant pour celle-ci, sélectionné dans le groupe consistant en des anticorps polyclonaux ou monoclonaux, ou des parties de ceux-ci, ou des acides nucléiques.

2. Procédé selon la revendication 1, où la biopsie est prélevée à partir d'un vaisseau sanguin.

3. Procédé selon la revendication 1 ou 2, où l'agent de liaison est un anticorps polyclonal ou monoclonal, ou une partie de ceux-ci.

4. Procédé selon la revendication 1 ou 2, où l'agent de liaison est un acide nucléique.

5. Procédé pour détecter une plaque d'athérosclérose chez un mammifère, le procédé comprenant les étapes consistant à
a) déterminer la quantité de chaîne alpha 10 de l'intégrine chez un mammifère *in vitro,*
b) attribuer un score à la quantité de chaîne alpha 10 de l'intégrine chez ledit mammifère, par rapport à un contrôle,
c) corréler la quantité obtenue à l'étape b) ci-dessus avec des quantités obtenues à partir du contrôle afin de détecter ladite plaque d'athérosclérose chez le mammifère,
où la détermination de la quantité de chaîne alpha 10 de l'intégrine consiste en outre à mettre en contact la chaîne alpha 10 de l'intégrine avec un agent de liaison ayant un site de liaison spécifique pour ladite chaîne alpha 10 de l'intégrine, ou pour un ARN codant pour celle-ci, sélectionné dans le groupe consistant en des anticorps polyclonaux ou monoclonaux, ou des parties de ceux-ci, ou des acides nucléiques.

6. Procédé selon la revendication 5, où l'agent de liaison est un anticorps polyclonal ou monoclonal, ou une partie de ceux-ci.

7. Procédé selon la revendication 5, où l'agent de liaison est un acide nucléique.

8. Procédés selon l'une quelconque des revendications 5 à 7, où le mammifère est un humain.

9. Procédés selon l'une quelconque des revendications 5 à 7, où le mammifère est une souris.

10. Utilisation d'une chaîne alpha 10 de l'intégrine en tant que marqueur diagnostique pour détecter une plaque d'athérosclérose *in vitro.*

11. Utilisation d'un agent de liaison ayant un site de liaison spécifique pour ladite chaîne alpha 10 de l'intégrine, ou pour un ARN codant pour celle-ci, dans la préparation d'un agent diagnostique pour détecter une plaque d'athérosclérose chez un mammifère, où l'agent de liaison est sélectionné dans le groupe consistant en des anticorps polyclonaux ou monoclonaux, ou des parties de ceux-ci, ou des acides nucléiques.

12. Utilisation selon la revendication 11, où l'agent de liaison est un anticorps polyclonal ou monoclonal, ou une partie de ceux-ci.

13. Utilisation selon la revendication 11, où l'agent de liaison est un acide nucléique.

14. Utilisation selon l'une quelconque des revendications 11 à 13, où le mammifère est un humain.

15. Utilisation selon l'une quelconque des revendications 11 à 13, où le mammifère est une souris.
